# EUROPÄISCHE PATENTANMELDUNG

(11) **EP 0 751 135 A1**
(43) Veröffentlichungstag der Anmeldung: **02.01.1997**
(21) Anmeldenummer: 96110143.3
(22) Anmeldetag: 24.06.1996
(51) Int. Cl.: C07D 301/12, C07D 301/02, C07D 301/03, C07D 303/02

(54) **Verfahren zur Herstellung von chiralen Epoxiden mit chiralen Mangantriazanonankomplexen als Oxidationskatalysatoren**

(30) Priorität: 30.06.1995 DE 19523890
(71) Anmelder: HOECHST AKTIENGESELLSCHAFT, 65929 Frankfurt am Main (DE)
(72) Erfinder: Beller, Matthias, Prof. Dr., 85737 Ismaning (DE); Tafesh, Ahmed, Dr., 65779 Kelkheim (DE); Fischer, Richard Walter, Dr., 65812 Bad Soden (DE); Scharbert, Bernd, Dr., 65934 Frankfurt (DE)

(57) **Zusammenfassung**

Die Erfindung betrifft ein Verfahren zur Herstellung von Epoxiden der Formel (1) 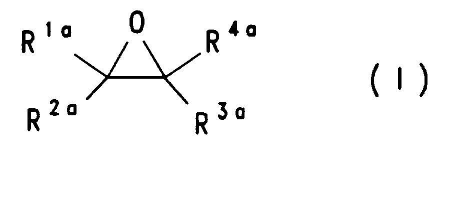 durch Umsetzung von Olefinen der Formel (II) 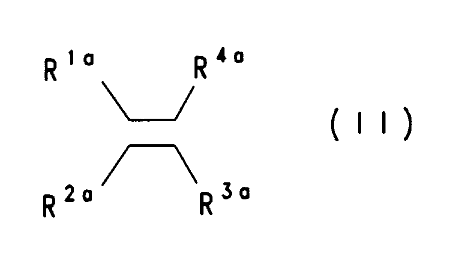 mit einem Oxidationsmittel, dadurch gekennzeichnet, daß man als Katalysator eine chirale Manganverbindung der allgemeinen Formel (III) einsetzt,

[Mnᵤ(L)ᵥ(OR)_{w}(µO)x(µOAc)µ]X_{z} (III)

worin bedeuten:
u, v gleich 1 oder 2;
w, x, y gleich 0, 1, 2 oder 3;
z gleich 1, 2 oder 3
mit der Maßgabe, daß, wenn u = 1 ist, v = 1, w = 1, 2, z = 1, 2, 3 sind, oder,
wenn u = 2 ist, v = 2, w = 0, 1, x = 1, y = 2, z = 1, 2 sind, oder
v = 2, w = 0, 1, x = 1, y = 2, z = 2, 3 sind, oder
v = 2, w = 0, 1, x = 3, y = 0, z = 1, 2 sind;
- R: (C₁-C₁₂)Alkyl,
- X: PF₆^{⊖}, F^{⊖}, Cl^{⊖}, Br^{⊖}, I^{⊖}, (C₆H₅)B^{⊖}, ClO₄^{⊖} bedeutet und
- L: für einen chiralen organischen Triazanonan-Liganden der allgemeinen Formel (II) steht.

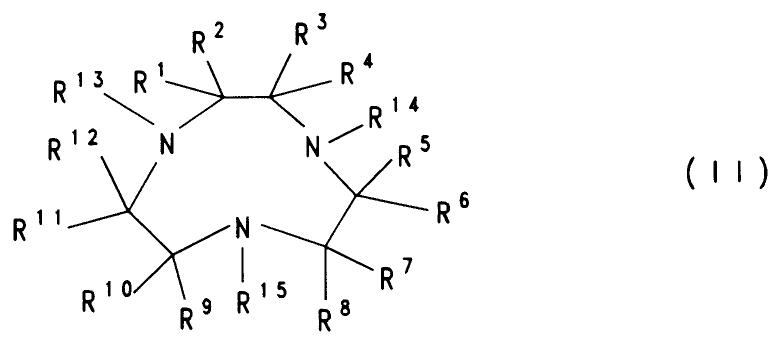

## Beschreibung

Stereoselektive Oxidationsreaktionen sind von zentraler Bedeutung für die Synthese einer großen Anzahl von Wirkstoffen und Wirkstoffzwischenprodukten für Pharmaka und Agrochemikalien. Insbesondere Epoxidationsreaktionen sind eine wichtige Methode zur Darstellung von chiralen Verbindungen aufgrund der vielfältigen Funktionalisierbarkeit der Produkte.

Bisher gibt es kein generelles Verfahren, das Epoxide gleichzeitig in hohen Ausbeuten und hohen Enantioselektivitäten zugänglich macht. Beste derzeit existierende Katalysatorsysteme zur enantioselektiven Epoxidation von Olefinen zu Epoxiden sind chirale Mangan-Salen-Komplexe, die zuerst von Jacobsen et al. (J. Am. Chem. Soc. 1990, 112, 2801; J. Am. Chem. Soc. 1991, 113, 7063) beschrieben wurden und später von Katsuki et al. (Synlett, 1993, 641; Tetrahedron Lett. 1990, 31, 7345) modifiziert wurden.
Die Klasse der Mangan-Salen-Katalysatoren ist für einen industriellen Einsatz nicht allgemein geeignet.
Ein großer Nachteil der beschriebenen Mn-Salen-Komplexe ist die lediglich für cis-Olefine befriedigende Selektivität der Reaktion. Bei allen anderen Klassen von Olefinen ist die Enantioselektivität technisch unzureichend für eine Nutzung.

Weiterhin ist es insbesondere aus technischer Sicht unzureichend, daß alle bisher bekannten Katalysatorsysteme nur außerordentlich schlechte Katalysator-Turn-Over Zahlen besitzen, so daß große Mengen an Katalysator - in der Regel zwischen 5-10 mol% - eingesetzt werden müssen.

Aus den genannten Gründen bestand ein großes industrielles Interesse bessere Verfahren für die enantioselektive Oxidation von Olefinen zu finden, das die beschriebenen Nachteile zur Herstellung von chiralen Epoxiden vermeidet und das Epoxide in hohen Ausbeuten und mit hohen Enantioselektivitäten zugänglich macht.

Die Aufgabe wird gelöst durch ein Verfahren zur Herstellung von Epoxiden der Formel (1) 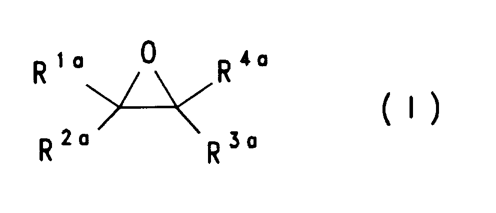 worin R^{1a} bis R^{4a} unabhängig voneinander Wasserstoff, (C₁-C₁₂)Alkyl, (C₃-C₁₂)Cycloalkyl, (C₆-C₁₂)Bicycloalkyl, (C₁-C₁₂)Alkenyl, (C₃-C₁₂)Cycloalkenyl, (C₁-C₁₂)Alkinyl, Alkoxy(C₁-C₁₂), O-Aryl, Aryl, Heteroaryl, NH(C₁-C₁₂)Alkyl, N(C₁-C₁₂)(Alkyl)₂, Halogen bedeuten, wobei R^{1a} und R^{2a}, R^{2a} und R^{3a}, R^{2a} und R^{4a} oder R^{3a} und R^{4a} auch zusammen einen Ring bilden können, durch Umsetzung von Olefinen der Formel (II) 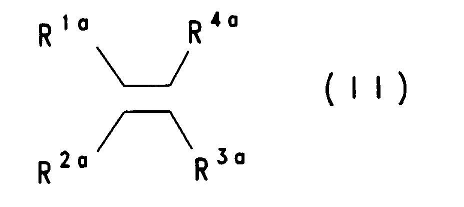 worin R^{1a} bis R^{4a} die angegebene Bedeutung besitzen, mit einem Oxidationsmittel, dadurch gekennzeichnet, daß man als Katalysator eine chirale Manganverbindung der allgemeinen Formel (III) einsetzt,

[Mnᵤ(L)ᵥ(OR)_{w}(µO)x(µOAc)µ]X_{z} (III)

worin bedeuten:
u, v gleich 1 oder 2;
w, x, y gleich 0, 1, 2 oder 3;
z gleich 1, 2 oder 3
mit der Maßgabe, daß, wenn u = 1 ist, v = 1, w = 1, 2, z = 1, 2, 3 sind, oder,
wenn u = 2 ist, v = 2, w = 0, 1, x = 1, y = 2, z = 1, 2 sind, oder
v = 2, w = 0, 1, x = 1, y = 2, z = 2, 3 sind, oder
v = 2, w = 0, 1, x = 3, y = 0, z = 1,2 sind;
- R: (C₁-C₁₂)Alkyl,
- X: PF₆^{⊖}, F^{⊖}, Cl^{⊖}, Br^{⊖}, I^{⊖}, (C₆H₅)B^{⊖}, ClO₄^{⊖} bedeutet und
- L: für einen chiralen organischen Triazanonan-Liganden der allgemeinen Formel (II) steht, 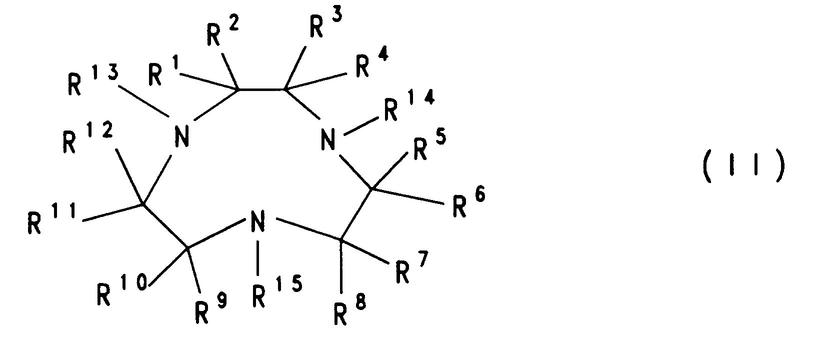
worin
R¹ bis R¹² unabhängig voneinander Wasserstoff, (C₁-C₁₂)Alkyl, (C₃-C₁₂)Cycloalkyl, (C₁-C₁₂)Alkenyl, (C₁-C₁₂)Alkoxy, (C₁-C₁₂)Acyloxy, Aryl, Heteroaryl, CH₂-Aryl, COOH, COO(C₁-C₁₂)Alkyl, COO-Aryl, CN, Halogen, C-(Halogen)₃, NH₂, NH-(C₁-C₁₂)-Alkyl, N(C₁-C₁₂-Alkyl)₂, NH-Aryl, N(Aryl)₂, N-Alkylaryl, S(C₁-C₁₂)Alkyl, SO(C₁-C₁₂)Alkyl, SO₂(C₁-C₁₂)Alkyl, P(C₁-C₁₂Alkyl)₂ und
R¹³ bis R¹⁵ Wasserstoff, (C₁-C₁₂)Alkyl, CH₂-Aryl, Aryl und Heteroaryl bedeuten.

Von Interesse ist das Verfahren zur Herstellung von Verbindungen der Formel (I) worin R^{1a} bis R^{4a} Wasserstoff, (C₁-C₆)Alkyl, (C₃-C₇)Cycloalkyl, (C₆-C₁₂)Bicycloalkyl, (C₁-C₆)Alkenyl, (C₃-C₇)Cycloalkenyl, Alkoxy-(C₁-C₁₂), Aryl, Heteroaryl oder das Pinan-Gerüst bedeutet. Für Aryl und Heteroaryl können z.B. Phenyl, Benzyl, Pyridyl, Naphthyl oder Bipysidyl stehen. Interessant ist beispielsweise die Herstellung von Verbindungen worin Formel (I) für folgende Strukturen steht: 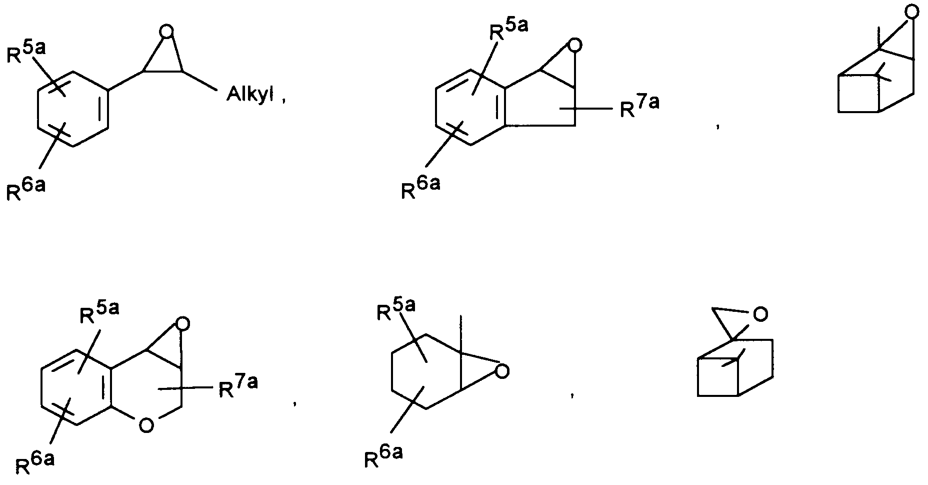 wobei R^{5a}, R^{6a}, R^{7a} Wasserstoff, (C₁-C₅)Alkyl, (C₁-C₆)Alkoxy oder Halogen bedeuten. Von besonderem Interesse ist das Verfahren zur Herstellung von Produkten der Formel (I) worin die Zielverbindung Styrolepoxid, substituierte Styrolepoxide wie 3-Chlorstyrolepoxid, trans-β-Methylstyrolepoxid, Indenepoxid, 3-Arylepoxyallylether wie p-(2-Methoxyethyl)phenyl]epoxyallylether oder Epoxychroman, substituierte Epoxychromane oder Cyclohexenepoxid oder Epoxypinan oder Carbonepoxid darstellt.

Wichtig sind insbesondere Verbindungen der Formel (I) worin 1 oder 2 Substituenten bevorzugt Wasserstoff darstellen und die restlichen Substituenten die oben angegebene Bedeutung besitzen.

Als Oxidationsmittel können Wasserstoffperoxid, Alkyl- oder Arylalkylhydroperoxide, Bisalkylperoxide, Luftsauerstoff in Gegenwart von Aldehyden, Alkali- oder Erdalkali-Metallhypochlorite oder -periodate, Aminoxide oder Persäuren eingesetzt werden. Gute Resultate liefern z.B. Wasserstoffperoxid, tert. Butylhydroperoxid, Bis-tert.-butylperoxid, Natriumhypochlorit, Natriumperiodat, Idosobenzol oder Pyridin-N-oxid.

Als Lösungsmittel finden generell inerte organische Lösungsmittel Verwendung, gut geeignet sind z.B. tert. Butanol, Methanol, Tetrahydrofuran, Toluol, Ethylacetat oder Essigsäure. Es ist auch möglich das Verfahren in Lösungsmittelgemischen oder Mehrphasensystemen durchzuführen. Die als Katalysator verwendeten Mangankomplexe sind in der am gleichen Tag eingereichten Anmeldung P ............... beschrieben.

Die eingesetzten Mangankatalysatoren werden in der Regel vor der eigentlichen Reaktion isoliert synthetisiert, sie können jedoch auch in bestimmten Fällen in situ erzeugt werden.

Das Verfahren wird im allgemeinen bei Temperaturen von 0 - 100°C durchgeführt. Bewährt haben sich Temperaturen von 20 bis 80°C, insbesondere 30 bis 60°C.

Es hat sich bewährt pro Doppelbindungsäquivalent Alken von 1,0 bis 2,0 Mol, insbesondere 1,05 bis 1,8, bevorzugt 1,1 bis 1,7 Mol Oxidationsmittel einzusetzen und bei einem pH-Wert von 1 bis 12, insbesondere 4 bis 10 zu arbeiten.

Die nachstehenden Beispiele dienen zur Erläuterung des erfindungsgemäßen Verfahrens, ohne es darauf zu beschränken.

### Allgemeine Arbeitsvorschriften

### 1) Herstellung der Oxidationslösungen:

100 ml tert.-Butanol werden mit 25 ml 30 %igem H₂O₂ (pro analysi-Qualität) versetzt und anschließend mit 30 g wasserfreiem NaSO₄ eine Stunde lang gerührt. Das Magnesiumsulfat wird dann abfiltriert. Die so erhaltene Oxidationslösung wird vorsorglich unter Kühlung (-10°C) aufbewahrt. Als alternative Oxidationsmittel zu Wasserstoffperoxid können, unter Erhalt vergleichbarer Umsatzzahlen, auch tert.-Butylhydroperoxid, Bis-tert.-butylperoxid (jeweils gelöst in Kohlenwasserstoffen, Natriumhypochlorit (NaOCl), N-Oxide (z.B. Pyridin-N-Oxid), Idosobenzol oder Periodate (z.B. NalO₄) verwendet werden.

### 2) Herstellung der Katalysatorlösungen

Zu 50 ml der oben beschriebenen Oxidationslösung werden jeweils 20 mmol der Katalysatoren A bis D gegeben. Nach Auflösen der katalytisch aktiven Verbindungen erhält man i.d.R. klare, manchmal gelblich gefärbte Lösungen die bei 0°C über lange Zeiträume haltbar sind. Bei dieser Temperatur ist die katalysatorbedingte Wasserstoffperoxid-Zersetzung nur gering (weniger als 0.5 % Zersetzung binnen 7 Tagen).

### 3) Katalytische Oxidation von Alkenen

Die nach den Punkten 1) bzw. 2) hergestellte Katalysatorlösung wird mit dem betreffenden Alken (s. Tabelle 1) versetzt; im Fall von reaktiven Olefinen (Cyclohexen, 2,3-Dimethylbuten-2) konnte eine Erwärmung der Reaktionsmischung festgestellt werden. Die Menge des eingesetzten Alkens entspricht bezüglich der Doppelbindungsäquivalente zwischen 60 und 90 % der Molmenge des mit der Katalysatorlösung vorgelegten Wasserstoffperoxids. I.d.R. war die Oxidationsreaktion nach 1 h beendet (Abnahme der Katalysatoraktivität). Oftmals hat es sich allerdings als günstig erwiesen, die Oxidationen über einen Zeitraum von ca. 18 h ggf. bei leicht erhöhter Temperatur (40 - 50°C) zu halten um die erzielten Umsätze zu erreichen. Zur Entfernung des noch vorhandenen Wasserstoffperoxids wird eine katalytische Menge Braunstein MnO₂ zu der Reaktionsmischung, unter kräftigem Rühren gegeben. Zur Entfernung des gebildeten Wassers wird nach ca. 30 min dann mit einer überstöchiometrischen Menge Na₂SO₄ versetzt, 5 - 10 min gerührt und über eine mit Celite (eingetragenes Warenzeichen der Fa. Manville Corp., Denver, USA) beschickte Glasfritte abfiltriert. Der Filterrückstand wird dreimal mit wenig THF gewaschen. Die vereinigten Filtrate werden anschließend von den Lösungsmitteln unter vermindertem Druck befreit. Die so erhaltenen Rohprodukte werden zur weiteren Reinigung entweder aus gängigen Lösungsmitteln umkristallisiert oder im Vakuum einer Quecksilberdiffusionspumpe (p < 0.5 mbar) möglichst schonend, d.h. ohne hohe thermische Belastung destilliert.

### pH-Wert der Oxidationsreaktionen:

Der pH-Wert der jeweilig zur Reaktion kommenden Katalysatorlösung kann - individuell abgestimmt auf die Bedürfnisse der zu gewinnenden Epoxide - über einen breiten Bereich variieren (von 1 bis 12), vorzugsweise arbeitet man jedoch im Bereich zwischen pH = 4 bis 10. In jedem Fall ist es günstig den Wassergehalt des Reaktionssystems niedrig zu halten (z.B. um eine unerwünschte Ringöffnung des Epoxides bzw. einen Aktivitätsverlust des Katalysators zu vermeiden).

Anwendungsbeispiele 1 bis 12 sind in Tabelle 1 aufgeführt, Tabelle 2 gibt die erhaltenen ee-Werte an.

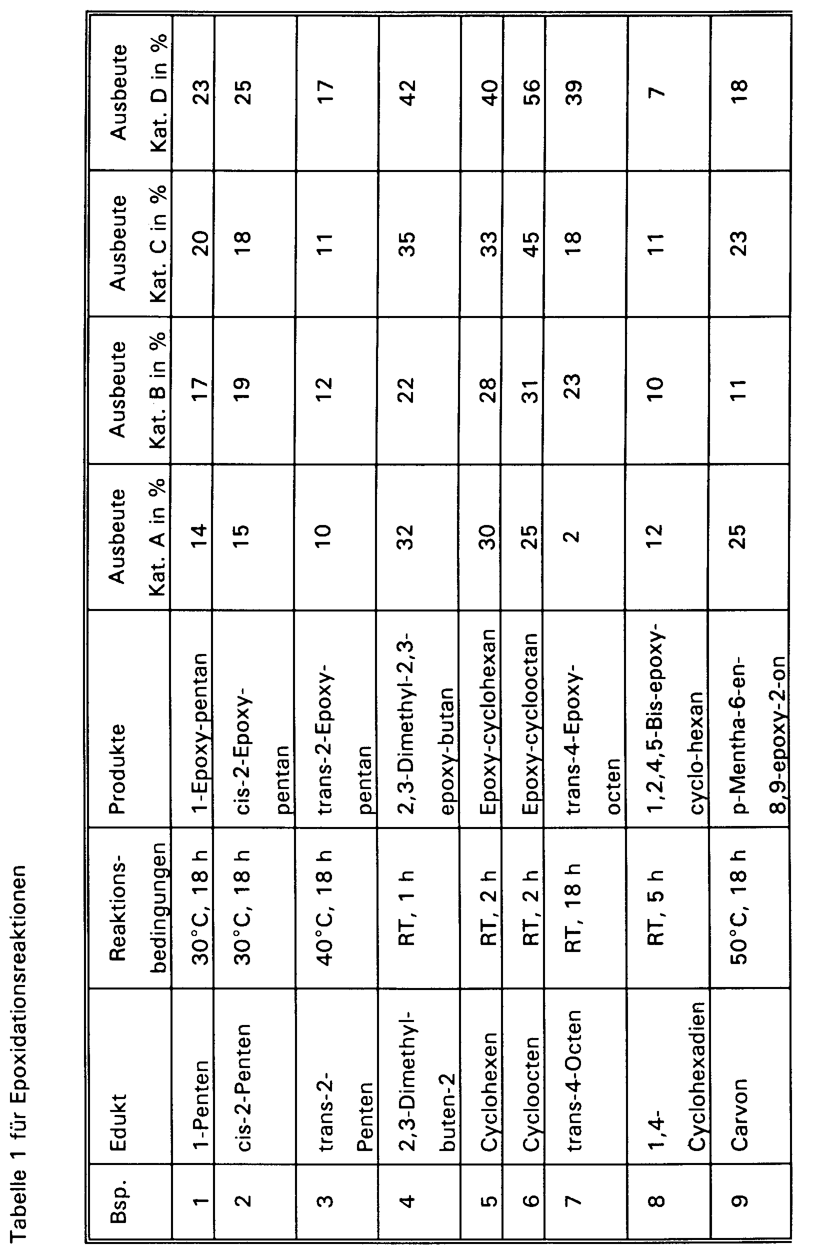

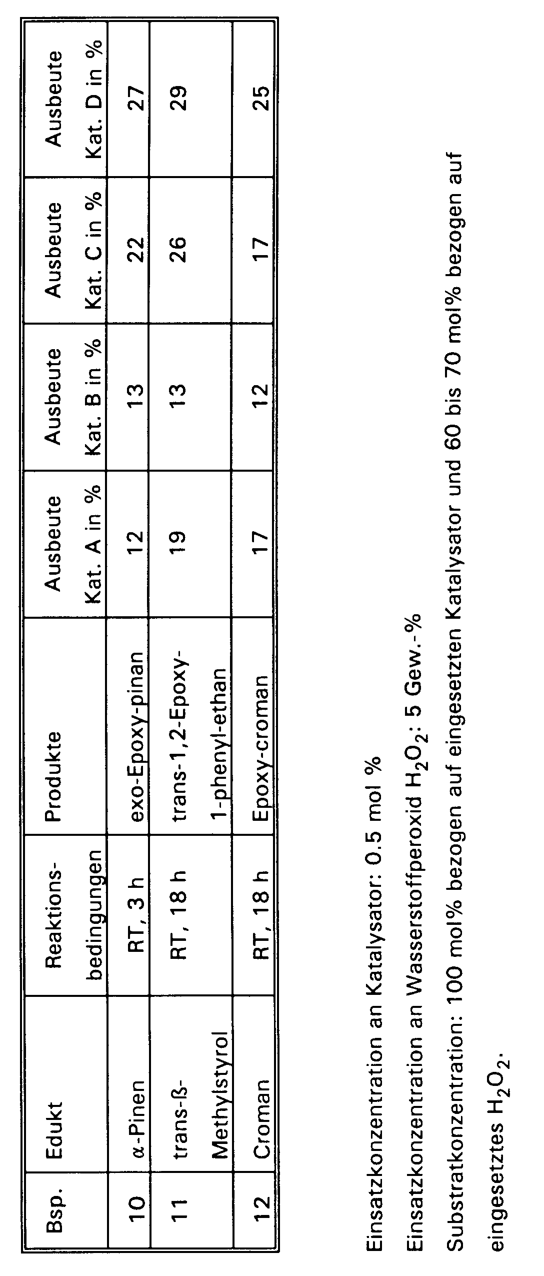

**Tabelle 2**

| zur enantioselektiven Epoxidation der mit den chiralen Katalysatoren A - D erzeugten optisch aktiven Epoxide: | | | | | | | |
|---|---|---|---|---|---|---|---|
| Bsp. | Edukt | Reaktionsbedingungen | Produkte | ee in % Kat A | ee in % Kat B | ee in % Kat C | ee in % Kat D |
| 1 | Carvon | RT, 16 h | p-Mentha-6-en-8,9-epoxy-2-on | 43 | 55 | 32 | 30 |
| 2 | α-Pinen | 5°C, 20 h | exo-Epoxy-pinan | 58 | 48 | 44 | 51 |
| 3 | trans-2-Penten | 40°C, 18 h | trans-2-Epoxy-pentan | 9 | 8 | 8 | 13 |
| 4 | Croman | 12°C, 16 h | Epoxy-croman | 72 | 78 | 68 | 80 * |
| 5 | 4-Methoxy-ethyl-phenyl-allylether | 20°C, 14 h | | 89 | 69 | 92 | 94 |

| | | | | | | | |
|---|---|---|---|---|---|---|---|
| * ee hier 85 bis 90 % wenn statt Wasserstoffperoxid NaOCl oder tert.-Butylhydroperoxid als Oxidationsmittel verwendet wurde. | | | | | | | |

## Patentansprüche

1. Verfahren zur Herstellung von Epoxiden der Formel (1) 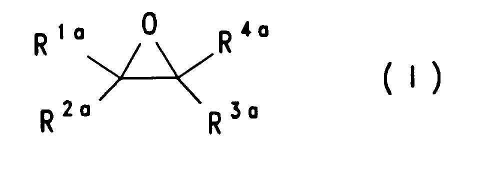 worin R^{1a} bis R^{4a} unabhängig voneinander Wasserstoff, (C₁-C₁₂)Alkyl, (C₃-C₁₂)Cycloalkyl, (C₆-C₁₂)Bicycloalkyl, (C₁-C₁₂)Alkenyl, (C₃-C₁₂)Cycloalkenyl, (C₁-C₁₂)Alkinyl, Alkoxy(C₁-C₁₂), O-Aryl, Aryl, Heteroaryl, NH(C₁-C₁₂)Alkyl, N(C₁-C₁₂)(Alkyl)₂, Halogen bedeuten, wobei R^{1a} und R^{2a}, R^{2a} und R^{3a}, R^{2a} und R^{4a} oder R^{3a} und R^{4a} auch zusammen einen Ring bilden können, durch Umsetzung von Olefinen der Formel (II) 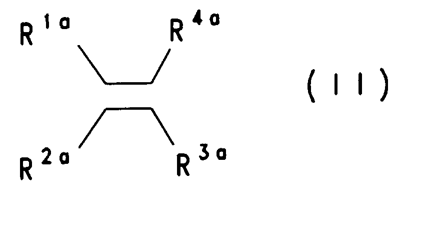 worin R^{1a} bis R^{4a} die angegebene Bedeutung besitzen, mit einem Oxidationsmittel, dadurch gekennzeichnet, daß man als Katalysator eine chirale Manganverbindung der allgemeinen Formel (III) einsetzt,
[Mnᵤ(L)ᵥ(OR)_{w}(µO)x(µOAc)µ]X_{z} (III)
worin bedeuten:
u, v gleich 1 oder 2;
w, x, y gleich 0, 1, 2 oder 3;
z gleich 1, 2 oder 3
mit der Maßgabe, daß, wenn u = 1 ist, v = 1, w = 1, 2, z = 1, 2, 3 sind, oder,
wenn u = 2 ist, v = 2, w = 0, 1, x = 1, y = 2, z = 1, 2 sind, oder
v = 2, w = 0, 1, x = 1, y = 2, z = 2, 3 sind, oder
v = 2, w = 0, 1, x = 3, y = 0, z = 1, 2 sind;
R (C₁-C₁₂)Alkyl,
X PF₆^{⊖}, F^{⊖}, Cl^{⊖}, Br^{⊖}, I^{⊖}, (C₆H₅)B^{⊖}, ClO₄^{⊖} bedeutet und
L für einen chiralen organischen Triazanonan-Liganden der allgemeinen Formel (II) steht, 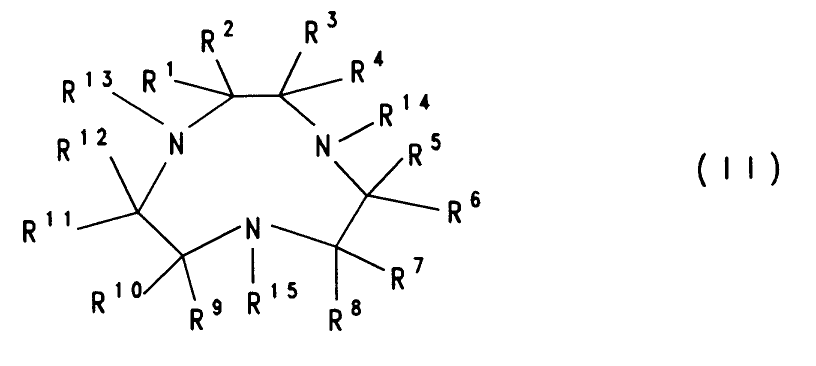
worin
R¹ bis R¹² unabhängig voneinander Wasserstoff, (C₁-C₁₂)Alkyl, (C₃-C₁₂)Cycloalkyl, (C₁-C₁₂)Alkenyl, (C₁-C₁₂)Alkoxy, (C₁-C₁₂)Acyloxy, Aryl, Heteroaryl, CH₂-Aryl, COOH, COO(C₁-C₁₂)Alkyl, COO-Aryl, CN, Halogen, C-(Halogen)₃, NH₂, NH-(C₁-C₁₂)-Alkyl, N(C₁-C₁₂-Alkyl)₂, NH-Aryl, N(Aryl)₂, N-Alkylaryl, S(C₁-C₁₂)Alkyl, SO(C₁-C₁₂)Alkyl, SO₂(C₁-C₁₂)Alkyl, P(C₁-C₁₂Alkyl)₂ und
R¹³ bis R¹⁵ Wasserstoff, (C₁-C₁₂)Alkyl, CH₂-Aryl, Aryl und Heteroaryl bedeuten.

2. Verfahren nach Anspruch 1, wobei R^{1a} bis R^{4a} unabhängig voneinander Wasserstoff, (C₁-C₆)Alkyl, (C₃-C₇)Cycloalkyl, (C₆₋C₁₂)Bicycloalkyl, (C₁-C₆)Alkenyl, (C₃-C₇)Cycloalkenyl, (C₁-C₆)Alkoxy, Phenyl, das auch mit Halogen, (C₁-C₆)Alkyl, (C₁-C₆)Alkoxy substituiert sein kann, bedeutet.

3. Verfahren nach Anspruch 1 oder 2, wobei 1 oder 2 der Substituenten R^{1a} bis R^{4a} Wasserstoff bedeuten und die restlichen die angegebene Bedeutung besitzen.

4. Verfahren nach Anspruch 1, dadurch gekennzeichnet, daß Formel (I) für folgende Strukturen steht: 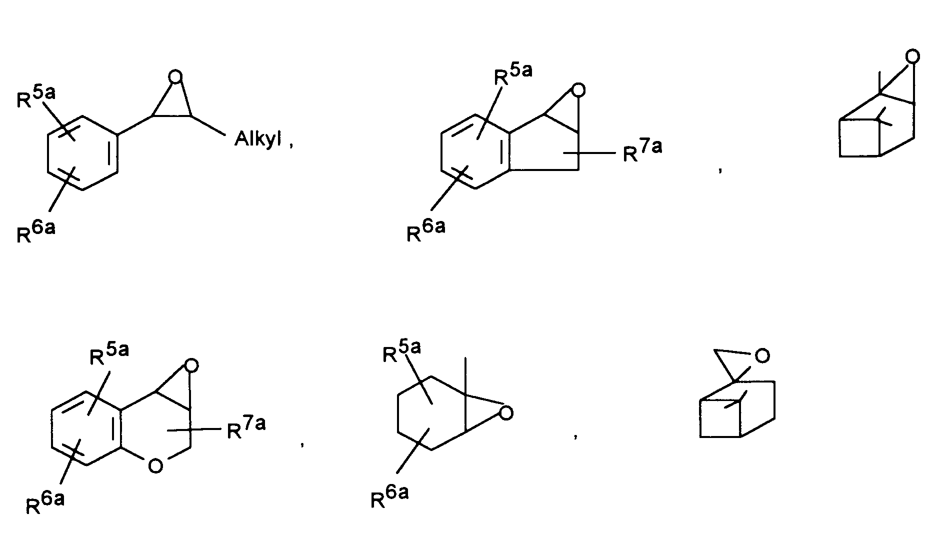 wobei R^{5a}, R^{6a}, R^{7a} Wasserstoff, (C₁-C₅)Alkyl, (C₁-C₆)Alkoxy oder Halogen bedeuten.

5. Verfahren nach Anspruch 1, dadurch gekennzeichnet, daß Formel (I) für Styrolepoxid, 3-Chlorstyrolepoxid, trans-β-Methylstyrolepoxid, Indenepoxid, p-(2-Methoxyethyl)phenyl]epoxyallylether, Epoxychroman, Cyclohexenepoxid, Epoxypinan oder Carbonepoxid steht.

6. Verfahren nach mindestens einem der Ansprüche 1 bis 5, dadurch gekennzeichnet, daß als Oxidationsmittel Wasserstoffperoxid, Alkyl-oder Arylalkylhydroperoxide, Bisalkylperoxide, Luftsauerstoff in Gegenwart von Aldehyden, Alkali- oder Erdalkalimetallhypochlorite oder -periodate, Aminoxide oder Persäuren eingesetzt werden.

7. Verfahren nach mindestens einem der Ansprüche 1 bis 6, dadurch gekennzeichnet, daß als Oxidationsmittel Wasserstoffperoxyd, tert. Butylhydroperoxid, Bis-tert.-butylperoxid, Natriumhypochlorit, Natriumperiodat, Idosobenzol oder Pyridin-N-oxid eingesetzt wird.

8. Verfahren nach mindestens einem der Ansprüche 1 bis 7, dadurch gekennzeichnet, daß als Lösungsmittel inerte organische Lösungsmittel, insbesondere tert. Butanol, Methanol, Ethylacetat, Tetrahydrofuran, Toluol oder Essigsäure eingesetzt wird.

9. Verfahren nach mindestens einem der Ansprüche 1 bis 8, dadurch gekennzeichnet, daß pro Doppelbindungsäquivalent Alken von 1,0 bis 2,0, insbesondere 1,05 bis 1,8, bevorzugt 1,1 bis 1,7 Mol Oxidationsmittel eingesetzt werden.

10. Verfahren nach mindestens einem der Ansprüche 1 bis 9, dadurch gekennzeichnet, daß die Reaktion bei einer Temperatur von 0 bis 100°C, insbesondere 20 bis 80°C, bevorzugt 30 bis 60°C durchgeführt wird.

11. Verfahren nach mindestens einem der Ansprüche 1 bis 10, dadurch gekennzeichnet, daß bei einem pH-Wert von 1 bis 12, insbesondere 4 bis 10 gearbeitet wird.
